# EUROPEAN PATENT APPLICATION

(11) **EP 3 228 264 A1**
(43) Date of publication of application: **11.10.2017**
(21) Application number: 14907443.7
(22) Date of filing: 05.12.2014
(51) Int. Cl.: A61B 17/135

(54) **TOURNIQUET**

(71) Applicant: Daesung Maref Co., Ltd, Gunpo-si, Gyeonggi-do 435-862 (KR)
(72) Inventor: LEE, Jae Hwa, Seoul 156-769 (KR); HA, Jung Hun, Suwon-si Gyeonggi-do 440-707 (KR)
(74) Representative: Vossius & Partner Patentanwälte Rechtsanwälte mbB
(86) International application number: PCT/KR2014/011923
(87) International publication number: WO 2016/088924

(57) **Abstract**

Provided is a tourniquet including an internal expansion and contraction portion that includes a gas injection and discharge portion to allow injection and discharge of a gas and expands or contracts according the injection and discharge of the gas and an external accommodation portion that has an incision in a longitudinal direction at one surface to have an accommodation space in which the internal expansion and contraction portion is able to be accommodated.

## Description

### [Technical Field]

The present invention relates to a tourniquet capable of separating an internal expansion and contraction portion and an external accommodation portion for water-washing or water-cleaning and putting one or both of the internal expansion and contraction portion and the external accommodation portion into an autoclave for sterilization and disinfection, which is impossible for a conventional tourniquet.

### [Background Art]

A tourniquet is a medical device for stopping a bleeding part bleeding.

As an example of the tourniquet, Korean Patent Publication No. 10-2012-0098766 (published on September 5, 2012) discloses a tourniquet. In a specification of the corresponding invention, it is disclosed that the present invention relates to first aid articles, and more particularly, to tourniquets and one embodiment of the claimed subject matter provides a tourniquet article having a substantially rigid base. A handle is provided, and the handle has first and second ends and a slot disposed between the first and second ends. A strap is disposed adjacent to the substantially rigid base and passes through the slot of the handle. A structural member having first and second sides is attached to the base at least at first and second points along each of the first and second sides of the base such that the strap is sandwiched between at least a portion of the structural member and the base.

Meanwhile, Korean Utility Model Publication No. 20-2013-0000081 (published on January 3, 2013) discloses a tourniquet apparatus. In a specification of the corresponding model, it is disclosed that the present design includes two components such as a tourniquet pressurizing band 250 and a tourniquet supporting member 350, the tourniquet supporting member 350 includes an upper end fixing band 354 for fixing an upper end of a hand to the supporting member 350, a lower end fixing band 356 for fixing a lower end of a wrist to the supporting member 350, and a pressurizing band 350 (sic) to which a pressurizing portion that pressurizes an opening of a catheter is attached, the pressurizing band 350 (sic) includes pressurizing band slit grooves 264 formed on left and right of a central lower end of the supporting member 350 to allow the pressurizing band to pass through the two slit grooves and be fixed to the supporting member, and the pressurizing band 350 (sic) includes a pressurizing portion group that rotates a male screw height adjustor 252 and moves a pressurizing male screw 254 integrated with a pressurizing portion 256 downward to allow the pressurizing portion 256 to pressurize the opening of the catheter.

When conventional tourniquets including the above-described tourniquets are sterilized for sterilization or washing after being used, a sterilizing gas (E/O gas) that is injurious to human bodies is used herein. Due to use of the above-described sterilizing gas injurious to human bodies, it was apprehended that a person in charge of sterilization in a clinic or a hospital was damaged.

Also, conventional tourniquets have a problem in which a stronger pressure is injected to prevent a phenomenon in which bleeding is not stopped well due to a pinch (that means an inconsistent part of an air tube) when a pneumatic pressure is injected into a conventional tourniquet in such a way that a corresponding part has a bruise due to the pressure for stopping the bleeding and the bruise continues for several weeks or more.

However, in conventional technologies, basic awareness of the problem of tourniquets caused by the occurrence of pinches is not present.

The present applicant(s) has(have) newly recognized that the above-described occurrence of pinches is a fundamental issue of tourniquets and it is necessary to develop new tourniquets for overcoming the issue of tourniquets.

### Prior Documents

### Patent Documents

(Patent Document 1) Korean Patent Publication No. 10-2012-0098766 (published on September 5, 2012 and titled Tourniquet)
(Patent Document 2) Korean Utility Model Publication No. 20-2013-0000081 (published on January 3, 2013 and titled Tourniquet apparatus)

### [Disclosure of Invention]

### [Technical Problem]

It is an aspect of the present invention to provide a tourniquet including an internal expansion and contraction portion and an external accommodation portion that are separable for disinfection, sterilization, and washing.

It is another aspect of the present invention to provide a tourniquet including an internal expansion and contraction portion and an external accommodation portion, one or both thereof available for being put into an autoclave for steam sterilization to be sterilized or disinfected, which is impossible for a conventional tourniquet.

### [Technical Solution]

One aspect of the present invention provides a tourniquet including an internal expansion and contraction portion that includes a gas injection and discharge portion to allow injection and discharge of a gas and expands or contracts according the injection and discharge of the gas and an external accommodation portion that has an incision in a longitudinal direction at one surface to have an accommodation space in which the internal expansion and contraction portion is able to be accommodated.

The internal expansion and contraction portion may be formed of a resin.

The gas injection and discharge portion may include an attachment portion connected to a hole formed in the internal expansion and contraction portion while covering and sealing the hole, a gas injection and discharge tube connected to the attachment portion to inject or discharge a gas, and a stopper that opens and closes an end of the gas injection and discharge tube.

One or more of the internal expansion and contraction portion, the attachment portion, the gas injection and discharge tube, and the stopper may be injection-molded.

A hole may be formed at the incision of the external accommodation portion corresponding to the gas injection and discharge tube.

A tourniquet fixing extension band for surrounding and fixing a bleeding part of a user who uses the tourniquet to stop the bleeding may be formed at one end of the external accommodation portion.

The tourniquet fixing extension band may include a Velcro component separately or in connection with the external accommodation portion and may surround and fix the bleeding part of the user who uses the tourniquet to stop the bleeding.

A tourniquet fixing string for fixing the tourniquet by surrounding the bleeding part of the user who uses the tourniquet may be formed at the other end of the external accommodation portion.

At least one of one surface and the other surface of the external accommodation portion may be formed of a cotton material having elasticity in a longitudinal direction.

The internal expansion and contraction portion and the external accommodation portion may be formed of a material capable of preventing the occurrence of pinches and may have a circular shape when the user wears the tourniquet.

One or more of the internal expansion and contraction portion, the attachment portion, the gas injection and discharge tube, the stopper, and the external accommodation portion may be formed of a material capable of enduring a temperature of 135 °C or more.

One or more of the internal expansion and contraction portion, the attachment portion, the gas injection and discharge tube, the stopper, and the external accommodation portion may be formed of a material capable of enduring high pressure steam sterilization, autoclave sterilization, gamma sterilization, E/O gas sterilization, plasma sterilization, and dry heat sterilization.

### [Advantageous Effects]

In a tourniquet according to embodiments of the present invention, first, there is provided an advantage of separating an internal expansion and contraction portion and an external accommodation portion from each other for disinfection, sterilization, and washing.

Second, there is provided an advantage of putting one or both of the internal expansion and contraction portion and the external accommodation portion into an autoclave for steam sterilization to sterilize or disinfect them, which is impossible for a conventional tourniquet.

Third, since sterilization and disinfection are easily performable using an autoclave, it is possible to minimize time or human power consumption of a person in charge in a clinic or a hospital.

Fourth, an economic effect may be expected by reducing human power consumption.

Fifth, since sterilization and disinfection of the tourniquet are simply and completely performable using the autoclave and the internal expansion and contraction portion and the external accommodation portion are separable to be water-washed, it is possible to minimize a concern of causing secondary infection or contagion of a user who uses the corresponding tourniquet.

Sixth, it is possible to solve a problem in using a conventional tourniquet in which pinches frequently occur. That is, since the internal expansion and contraction portion are formed of a resin such as silicone, pinches that occur in a conventional tourniquet do not occur.

### [Brief Description of Drawings]

FIG. 1 is a view illustrating the whole configuration of a tourniquet according to an exemplary embodiment of the present invention,
FIG. 2 is a view illustrating a case in which a pinch occurs when wearing a conventional tourniquet, and
FIG. 3 is a view illustrating that a pinch does not occur when a user wears the tourniquet according to the exemplary embodiment of the present invention.

### [Mode for Invention]

Hereinafter, an exemplary embodiment of the present invention will be described with reference to the attached drawings. Before this, the terms used throughout the specification and the claims should not be limited to general or lexical meanings and should be understood as having meanings and concepts in accordance with the technical concept of the present disclosure based on the principle in which the inventor can adequately define the meanings of the terms to explain the present disclosure in the best way.

Accordingly, since configurations described and shown in the embodiment and drawings disclosed in the specification are merely the most exemplary one embodiment of the present invention and do not representative the entire technical concept of the present invention, it should be understood that various equivalents and modifications capable of replacing them may be present at the point in time of filing the present application.

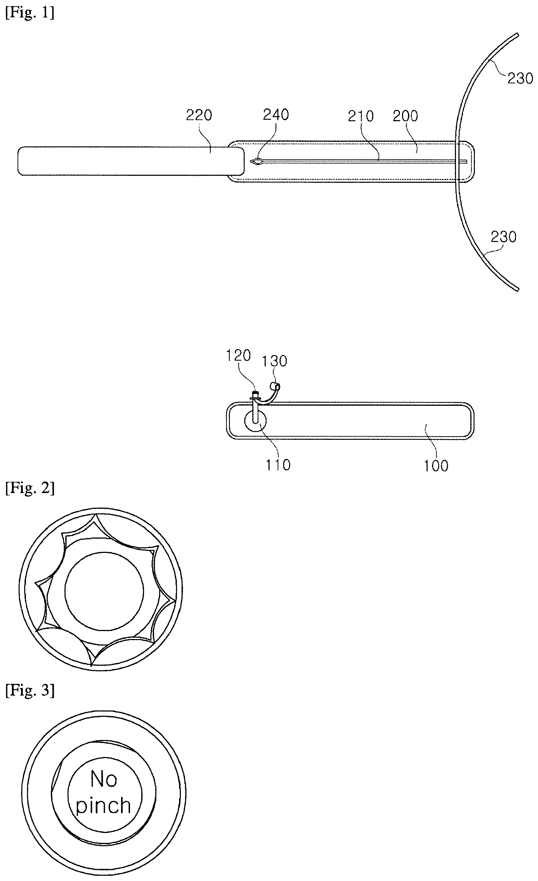

FIG. 1 is a view illustrating the whole configuration of a tourniquet according to an exemplary embodiment of the present invention.

As shown in FIG. 1, the tourniquet according to the present invention includes an internal expansion and contraction portion 100 and an external accommodation portion 200.

Here, the internal expansion and contraction portion 100 includes gas injection and discharge portions 110, 120, and 130 that allow a gas to be injected and discharged and expands or contracts according to injection and discharge of the gas.

The internal expansion and contraction portion 100 may be formed of a synthetic resin material such as silicone or a rubber resin to be expandable and contractible according to the injection and discharge of the gas.

Also, the gas injection and discharge portions may include an attachment portion 110 connected to a hole formed in the internal expansion and contraction portion while covering and sealing the hole, a gas injection and discharge tube 120 connected to the attachment portion to inject or discharge a gas, and a stopper 130 that opens and closes an end of the gas injection and discharge tube.

Also, one or more of the internal expansion and contraction portion, the attachment portion, the gas injection and discharge tube, and the stopper may be inj ection-molded.

That is, since the internal expansion and contraction portion 100 is formed of a resin such as silicone, components may be manufacturing through injection molding to improve productivity. The internal expansion and contraction portion 100 may be manufactured by overlapping a double-sided flat resin material formed through injection molding and connecting edges thereof.

As described above, since being formed of a resin such as silicone, the internal expansion and contraction portion 100 may have an advantage in which a pinch that occurs at a conventional tourniquet does not occur.

FIG. 3 illustrates a difference from a conventional tourniquet of FIG. 2. Additionally, when the external accommodation portion is also manufactured using a material capable of preventing the occurrence of pinches, it is possible to have an approximately circular shape when a user wears the tourniquet. Accordingly, it is possible to overcome a problem in which a stronger pressure is injected to prevent a phenomenon in which bleeding is not stopped well due to a pinch (that means an inconsistent part of an air tube) when a pneumatic pressure is injected into a conventional tourniquet in such a way that a corresponding part has a bruise due to the pressure for stopping the bleeding and the bruise continues for several weeks or more.

Also, the external accommodation portion 200 may have an incision 210 at one surface, and preferably, at a center of one surface in a longitudinal direction to have an accommodation space capable of accommodating the internal expansion and contraction portion 100.

Also, a hole 240 may be formed at the incision 210 of the external accommodation portion 200, corresponding to the gas injection and discharge tube 120.

Also, a tourniquet fixing extension band 220 for surrounding and fixing a bleeding part of a user who uses the tourniquet to stop the bleeding is formed at one end of the external accommodation portion 200.

Here, the tourniquet fixing extension band 220 has a Velcro component separately or in connection with the external accommodation portion and may easily surround the bleeding part of the user who uses the tourniquet to fix the tourniquet to stop the bleeding.

However, in addition to the Velcro component used herein, it is possible to fix using a buckle shape and various modifications and changes are available.

Also, a tourniquet fixing string 230 for fixing the tourniquet by surrounding the bleeding part of the user who uses the tourniquet may be formed at the other end of the external accommodation portion 200.

Also, at least one of one surface and the other surface of the external accommodation portion 200 may be formed of a cotton material having an elastic force in a longitudinal direction.

As described above, the internal expansion and contraction portion and the external accommodation portion are formed of a material capable of preventing the occurrence of pinches and may have an approximately circular shape when the user wears the tourniquet. Through this, it is available to have an overall uniform contact surface when the user wears the tourniquet.

Also, one or more of the internal expansion and contraction portion, the attachment portion, the gas injection and discharge tube, the stopper, and the external accommodation portion may be formed of a material capable of enduring high pressure steam sterilization, autoclave sterilization, gamma sterilization, E/O gas sterilization, plasma sterilization, and dry heat sterilization.

Also, one or more of the internal expansion and contraction portion, the attachment portion, the gas injection and discharge tube, the stopper, and the external accommodation portion may be formed of a material capable of enduring a temperature of 135 °C or more. That is, a material capable of enduring high pressure steam sterilization, autoclave sterilization, gamma sterilization, E/O gas sterilization, plasma sterilization, and dry heat sterilization may endure a temperature of 135 °C or more.

Through this, there are provided advantages in which the internal expansion and contraction portion and the external accommodation portion are separable to be disinfected, sterilized, and washed, and one or both of the internal expansion and contraction portion and the external accommodation portion may be put into an autoclave for steam sterilization to be sterilized and disinfected, which is unavailable for a conventional tourniquet.

In addition, since simple sterilization and disinfection using the autoclave are available, time and human power consumption of a person in charge in a clinic or a hospital may be minimized, a tourniquet may be simply and completely sterilized and disinfected through an autoclave, and the internal expansion and contraction portion and the external accommodation portion may be separated and washed using water to minimize a concern about causing secondary infection or contagion to a user using the corresponding tourniquet.

Although the present invention has been described through limited embodiments and drawings as described above, the present invention is not limited thereto and may be variously modified and changed by one of ordinary skill in the art without departing from the technical concept of the present invention and the scope of the following claims and equivalents thereof.

## Claims

1. A tourniquet comprising:
an internal expansion and contraction portion that comprises a gas injection and discharge portion configured to allow injection and discharge of a gas and expands or contracts according the injection and discharge of the gas; and
an external accommodation portion that has an incision in a longitudinal direction at one surface to have an accommodation space in which the internal expansion and contraction portion is able to be accommodated.

2. The tourniquet of claim 1, wherein the internal expansion and contraction portion is formed of a resin.

3. The tourniquet of claim 2, wherein the gas injection and discharge portion comprises:
an attachment portion connected to a hole formed in the internal expansion and contraction portion while covering and sealing the hole;
a gas injection and discharge tube connected to the attachment portion to inject or discharge gas; and
a stopper that opens and closes an end of the gas injection and discharge tube.

4. The tourniquet of claim 3, wherein one or more of the internal expansion and contraction portion, the attachment portion, the gas injection and discharge tube, and the stopper are injection-molded.

5. The tourniquet of claim 4, wherein a hole is formed at the incision of the external accommodation portion corresponding to the gas injection and discharge tube.

6. The tourniquet of claim 1, wherein a tourniquet fixing extension band for surrounding and fixing a bleeding part of a user who uses the tourniquet to stop the bleeding is formed at one end of the external accommodation portion.

7. The tourniquet of claim 6, wherein the tourniquet fixing extension band comprises a Velcro component separately or in connection with the external accommodation portion and is configured to surround and fix the bleeding part of the user who uses the tourniquet to stop the bleeding.

8. The tourniquet of claim 7, wherein a tourniquet fixing string for fixing the tourniquet by surrounding the bleeding part of the user who uses the tourniquet is formed at the other end of the external accommodation portion.

9. The tourniquet of claim 8, wherein at least one of one surface and another surface of the external accommodation portion is formed of a cotton material having elasticity in a longitudinal direction.

10. The tourniquet of claim 8, wherein the internal expansion and contraction portion and the external accommodation portion are formed of a material capable of preventing the occurrence of pinches and configured to have a circular shape when the user wears the tourniquet.

11. The tourniquet of claim 8, wherein one or more of the internal expansion and contraction portion, the attachment portion, the gas injection and discharge tube, the stopper, and the external accommodation portion are formed of a material capable of enduring a temperature of 135 °C or more.

12. The tourniquet of claim 8, wherein one or more of the internal expansion and contraction portion, the attachment portion, the gas injection and discharge tube, the stopper, and the external accommodation portion are formed of a material capable of enduring high pressure steam sterilization, autoclave sterilization, gamma sterilization, E/O gas sterilization, plasma sterilization, and dry heat sterilization.
